# EUROPEAN PATENT APPLICATION

(11) **EP 1 749 807 A1**
(43) Date of publication of application: **07.02.2007**
(21) Application number: 05016762.6
(22) Date of filing: 02.08.2005
(51) Int. Cl.: C07C 2/30, B01J 3/04

(54) **Method for producing linear alpha-olefins with improved product distribution**

(71) Applicant: Linde AG, 65189 Wiesbaden (DE); Saudi Basic Industries Corporation, Riyadh 11422 (SA)
(72) Inventor: Bölt, Heinz, 82515 Wolfratshausen (DE); Fritz, Peter Dr., 82008 Unterhaching (DE); Hoffmann, Karl-Heinz Dr., 82110 Germering (DE); Zander, Hans-Jörg Dr., 81479 München (DE); Musa, Fuad c/o Saudi Basic Industries Corporation, 11422 Riyadh (SA); Al Jasser, Turky c/o Saudi Basic Industries Corp., 11422 Riyadh (SA)
(74) Representative: Winkler, Andreas Fritz Ernst

(57) **Abstract**

The present invention relates to a method for preparing of linear alpha-olefins by oligomerizing of ethylene in the presence of an organic solvent and a homogenous catalyst comprising of a zirconium component and an organoaluminum component, characterized in that the method is carried out in more than one reactor being operated in parallel, wherein for each reactor the method conditions and parameters are independently adjustable.

## Description

The present invention relates to a method for preparing of linear alpha-olefins by oligomerizing of ethylene in the presence of an organic solvent and a homogenous catalyst comprising a zirconium component and an organoaluminum component.

Methods for the production of linear alpha-olefins are widely known in the art. Usually, a catalyst is utilized in that process comprising a zirconium component and an organoaluminum component which acts as an activator.

For example, DE 43 38 414 C1 discloses a process for the production of linear alpha-olefins, wherein one reactor is utilized into which a catalyst solution and ethylene are introduced. That process results in a product distribution of linear alpha-olefins having C₂-C₂₈ carbon atoms. It is already known in the field of the art that the product distribution may be varied by changing the ratio of the catalyst components, i.e. the ratio Al/Zr. The nature of the catalyst drives the distribution either to the light or heavy products.

However, even changing the Al/Zr ratio does not meet the market requirements in all cases. It is therefore an object of the present invention to provide a method for preparing of linear alpha-olefins which overcomes the drawbacks of the prior art. Especially, the method shall be provided showing improved product flexibility, especially with regard to product distribution.

This object is achieved in that the method is carried out in more than one reactor being operated in parallel, wherein for each reactor the method conditions and parameters are independently adjustable.

Preferably, wherein the Al/Zr ratio for the catalyst components is adjustable.

Additionally, temperature and pressure may be individually adjustable in each reactor.

In one embodiment, each reactor has at least one feed line for introducing the catalyst components, each dissolved in a solvent, into the reactor.

The catalyst components may be pre-mixed prior to introduction into the reactor.

Alternatively, each reactor has two feed lines to introduce the catalyst components separately into the reactor.

Most preferred, the outlet of some or all reactors is blended.

Additionally, the amount and rate of introduction of catalyst and/or catalyst components are controlled by a control device.

Preferably, the zirconium component has the formula ZrCl₄₋ₘXₘ, wherein X = OCOR or OSO₃R' with R and R' being independently alkyl, alkene and phenyl, and wherein 0 < m < 4.

Finallly, the organoaluminum component may be Al(C₂H₅)₃, Al₂Cl₃(C₂H₅)₃ or AlCl(C₂H₅)₂.

Surprisingly, it was found that the production flexibility may be increased and the product distribution may be adjusted according to market requirements, if a method is utilized wherein more than one reactor is operated in parallel, wherein for each reactor the method conditions and parameters may be adjusted independently from the other ones. Thus, the product distribution of each available reactor is adjusted individually, so that the required overall distribution can be met and market requirements may be fulfilled. As a further advantage of the method according to the present invention, the production quantity of specific products can be maintained even during shutdown of one reactor (e.g. for maintenance and cleaning) by adjustment of the reactors remaining on stream.

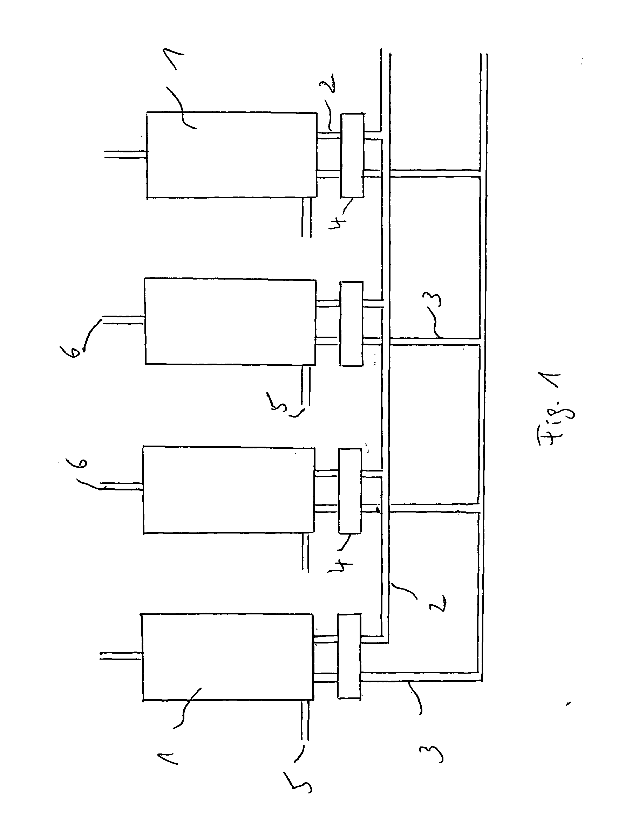

Additional advantages and features of the inventive method are further illustrated with reference to the accompanying drawing, wherein the figure illustrates a schematic diagram of a plant for the production of linear alpha olefins having four reactors to be operated in parallel.

According to the invention, the method for preparing of linear alpha-olefins is conducted in a plant having more than one reactor being operated in parallel. The figure shows four reactors 1. Into each reactor 1, one catalyst component, for example the zirconium component, may be introduced through a distribution line 2. A further distribution line 3 is provided to introduce the second catalyst component, for example the organoaluminum component, into each reactor. Thus, the zirconium component and organoaluminum component are mixed directly in the reactor. According to another embodiment of the invention, the catalyst components may be pre-mixed prior to introduction into each reactor, so that only one common distribution line is required. In a further embodiment (not shown), it is possible that each reactor is provided with separate lines for introducing the catalyst components, so that it is possible to introduce in each reactor different catalyst components, for example different organoaluminum compounds to influence the production flexibility.

For each reactor a control device 4 for controlling the amount and rate of the catalyst components to be introduced is additionally provided. Each control device 4 is thus employed to control all method conditions and parameters in each reactor 1, e.g. also temperature and pressure. Of course, only once control device 4 may be necessary to control all reactors. Each reactor is also provided with an ethylene feed line 5. Of course, the catalyst components are dissolved in a solvent, wherein toluene is preferably utilized.

Additionally, at least one discharge line 6 is provided for each reactor to discharge the reaction products. Someone skilled in the art is aware that several discharge lines are to be provided to remove both gaseous and liquid compounds from the reactor, which are omitted in the figure for the purpose of clarity. The discharge lines 6 of each reactor may combine so that the products obtained in the reactors may be mixed. As the reaction conditions and parameters may be adjusted in each reactor individually, the production flexibility and especially the product distribution may be optimized to fulfill market requirements.

The features disclosed in the foregoing description, in the claims and in the drawings may, both separately and in any combination thereof, be material for realizing the invention in diverse forms thereof.

## Claims

1. Method for preparing of linear alpha-olefins by oligomerizing of ethylene in the presence of an organic solvent and a homogenous catalyst comprising a zirconium component and an organoaluminum component, **characterized in that** the method is carried out in more than one reactor being operated in parallel, wherein for each reactor the method conditions and parameters are independently adjustable.

2. Method according to claim 1, wherein the Al/Zr ratio for the catalyst components is adjustable.

3. Method according to claim 1 or 2, wherein temperature and pressure are individually adjustable in each reactor.

4. Method according to any of the preceding claims, wherein each reactor has at least one feed line for introducing the catalyst components, each dissolved in a solvent, into the reactor.

5. Method according to any of the preceding claims, wherein the catalyst components are pre-mixed prior to introduction into the reactor.

6. Method according to claim 4, wherein each reactor has two feed lines to introduce the catalyst components separately into the reactor.

7. Method according to any of the preceding claims, wherein the outlet of some or all reactors is blended.

8. Method according to any of the preceding claims, wherein the amount and rate of introduction of catalyst and/or catalyst components are controlled by a control device.

9. Method according to any of the preceding claims, wherein the zirconium component has the formula ZrCl₄₋ₘXₘ, wherein X = OCOR or OSO₃R' with R and R' being independently alkyl, alkene and phenyl, and wherein 0 < m < 4.

10. Method according to any of the preceding claims, wherein the organoaluminum component is Al(C₂H₅)₃, Al₂Cl₃(C₂H₅)₃ or AlCl(C₂H₅)₂.
